# EUROPEAN PATENT APPLICATION

(11) **EP 2 022 514 A2**
(43) Date of publication of application: **11.02.2009**
(21) Application number: 08160578.4
(22) Date of filing: 17.07.2008
(51) Int. Cl.: A61M 1/00

(54) **Thoracic drainage device**

(30) Priority: 08.08.2007 IT MI20071649
(71) Applicant: Eurosets S.r.l., 41036 Medolla (MO) (IT)
(72) Inventor: Fontanili, Paolo, 42015, Correggio Re (IT); Paratelli, Ruggero, 41036, Medolla Mo (IT); Balugani, Fabio, 41032, Cavezzo Mo (IT)
(74) Representative: Modiano, Micaela Nadia

(57) **Abstract**

A thoracic draining device, comprising, in a monolithic structure, three interconnected contiguous chambers, which are adapted to be placed in partial vacuum by being connected to a source of suction which is capable of creating, within the device, a condition of vacuum which conveys away from the patient the exuded liquids and the air losses, said chambers comprising: a first chamber (100) for collecting the exuded liquids, which is adapted to be connected by means of a tube to the thoracic cavity of a patient, so as to receive said exuded liquids and the air losses that exit from said cavity, a second sealing chamber (200), provided at the bottom with a device for measuring said air losses, a third chamber (300) for adjusting the vacuum that occurs within the two preceding chambers, comprising a column of water (14) through which atmospheric air is made to pass which enters at the bottom formed by a perforated bubbling partition (15) and is conveyed thereat by a duct (16) after entering through a perforated plug (18) in order to be evacuated through a connector (8) which is designed to be connected to the source of suction, at the top of the third chamber (300), in said monolithic structure, a separator (23a, 23b) being provided for the two phases constituted by water and atmospheric air which are mixed in the stream that exits as a consequence of bubbling from the free surface of the water column (14) that is present in said third chamber (300), with consequent fall of the water onto said column (14) and exit of the atmospheric air from said chamber toward the suction source.

## Description

The invention relates to a thoracic drainage device.

Devices are known which are designed to be connected to the thoracic cavity of a patient after surgery, or a trauma, by means of a tube which conveys to the device exuded liquids and air losses that exit from such cavity, by way of the suction determined by the partial vacuum created within the device proper, or by gravity; these devices are indeed known as thoracic drainage devices.

Known devices are divided into three interconnected contiguous chambers, the first of which is the chamber for collecting the exuded liquids that originate, together with the air losses, from the patient and are intended to accumulate on the bottom of such chamber.

The second chamber is known as sealing chamber; it acts as a one-way valve, since it is capable of receiving the air losses that arrive from the first chamber, preventing their return, and is provided at the bottom with a device for measuring the air losses.

Finally, the third chamber is the chamber for adjusting the vacuum that occurs in the two preceding chambers as a consequence of connection to a source: its presence is necessary in order to ensure constancy of the value of the partial vacuum that is present in said chambers, in view of the fact that the suction source to which they are connected, constituted normally by a vacuum line of a general system, is subject to fluctuations.

Known devices are not free from disadvantageous drawbacks, and it is therefore the aim of the present invention to provide a thoracic drainage device which has improved characteristics from a functional standpoint and from a constructive standpoint.

This aim is achieved by a thoracic drainage device, characterized in that it comprises the features according to the appended claims.

Further characteristics and advantages of the present invention will become better apparent from the description of some preferred but not exclusive embodiments of the device according to the present invention, illustrated by way of non-limiting example in the accompanying drawings, wherein:
Figure 1 is a perspective view of the device according to the invention;
Figure 2 is a sectional view, taken along the line II-II of Figure 1;
Figure 3 is a view of a detail of the device according to the invention;
Figures 4 and 5 are views of a detail of Figure 2 in different functional conditions;
Figures 6 and 7 are views of two details of the device according to the invention;
Figures 8 and 9 are views of the detail of Figure 7 in two different functional conditions;
Figures 10 and 11 are views of two details according to further embodiments.

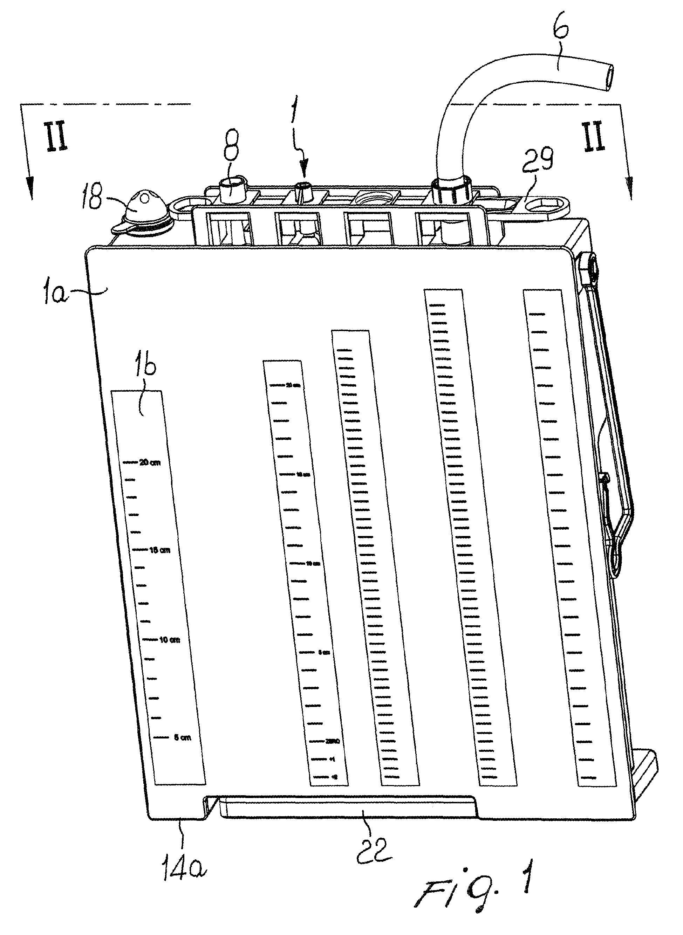

With reference to Figures 1 to 9, the reference numeral 1 generally designates a device, which comprises a first collection chamber 100, delimited by walls 2 and 3 and divided into three interconnected subchambers by partitions 4 and 5; the first collection chamber is designed to be connected by means of a tube 6 to the thoracic cavity of a patient, so as to receive the air losses along the arrow A and the exuded liquids along the arrow B in order to collect the latter at the bottom.

A second chamber 200, delimited by the wall 3 and by the wall formed by portions 7a, 7b, 7c, is designed to be connected by means of a connector 8 to a suction source and is connected by means of a connecting duct 9 to the first chamber 100, so that the same degree of vacuum that determines the suction of the exuded liquids and of the air losses occurs therein.

While, as mentioned, the exuded liquids are collected on the bottom of the first chamber 100, the air losses, following the arrows C and D, pass through the duct 9 and reach the second chamber 200, where they are directed by a partition 10, along the arrow E, toward the bottom in order to penetrate along the arrow F into a measurement device provided thereat, which is generally designated by the reference numeral 11 and is described in detail hereinafter; in output from the device 11 along the arrows G and H, which illustrate the crossing of a tap 12 comprised within the device, the air losses arrive along the arrow I at the connector 8 in order to be evacuated.

A third chamber 300 is delimited by the wall formed by the portions 7a, 7b, 7c and by a wall 13, and the adjustment of the vacuum that occurs in the two previously described chambers occurs therein in a known manner.

In the third chamber there is in fact a water column 14, which is typically 20 centimeters high and through which atmospheric air is made to pass by bubbling and penetrates along the arrow L at the bottom formed by a perforated bubbling partition 15, being conveyed thereto by a duct 16 formed by a partition 17 after entering the device through a plug 18 along the arrow M, to be then evacuated along the arrow N through the connector 8.

Since atmospheric pressure is present at the base of the water column 14, in a portion of space 19 at the top there is necessarily a partial vacuum whose value is equal to the height of the column, and is therefore typically equal to 20 centimeters of water column, and such partial vacuum, shared by the two previously described chambers, is absolutely constant.

Since a wall 1a of the device is provided with transparent regions 1b for viewing the content of the several chambers described above, a detailed description will follow with respect to the third chamber.

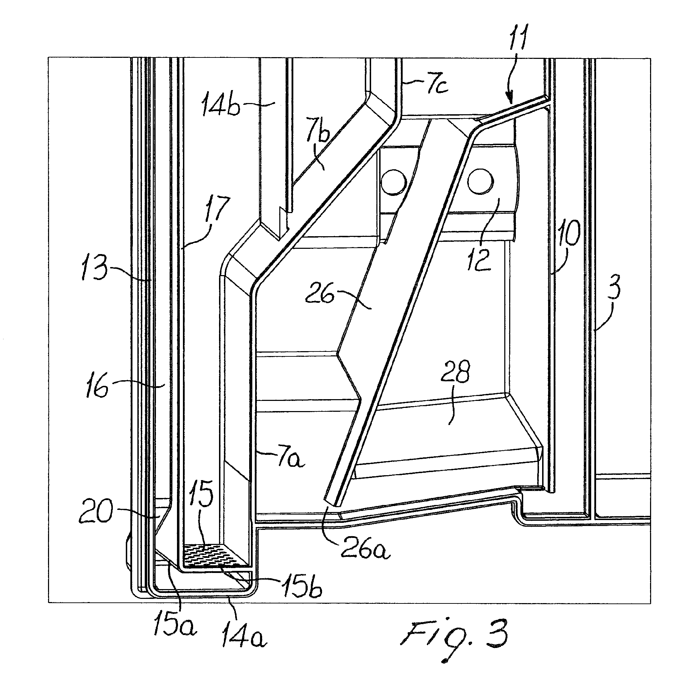

In order to reduce the noisiness of the device, as shown in Figure 3, a particular shaping has been provided for the duct 16, which conveys atmospheric air to the bubbling partition 15 provided monolithically with the structure of the device, by shaping the end portion of the bottom wall of the duct so as to form an inclined plane 20, thereby determining access of the atmospheric air to the bubbling partition 15 at the transverse side 15a thereof, which is much longer than the front side 15b.

One thus obtains an optimum subdivision of the atmospheric air in passing through the holes of the partition 15, with the formation of bubbles of minimum size and thus such as to ensure a good reduction of noisiness, which is directly proportional to the size of the bubbles.

Again with the goal of reducing the noisiness of the device, the sound waves in output have been subdivided by providing the plug 18 with four holes 18a, 18b, 18c, 18d. Moreover, the third chamber 300 of the device is provided with means which are capable of preventing the passage of water, due to intense bubbling of the atmospheric air in the water column 14, through a port 21 which connects the third chamber to the second chamber 200 and to the connector 8 of the suction source. From this standpoint, a base 14a of the device at the column 14 has been arranged so as to be coplanar with respect to the base of a support 22 of the device, thus obtaining the maximum spacing of the free surface of the column 14 from the port 21 without causing unwanted increases in the overall vertical space occupation of the device.

For the same purpose, at the top of the portion of space 19 there is a phase separator, which comprises a baffle 23a and a separator 23b; the two-phase stream determined by bubbling, which comprises water, represented by the black arrow, and atmospheric air, represented by the white arrow, enters through a port 24 the duct which is delimited by the two partitions and ends with a double opening: through a port 25a, the water descends toward the underlying column 14, separating from the current of atmospheric air that passes through a port 25b and immediately reaches, in a perfectly dry condition, the output port 21.

The presence of a partition 14b makes the described separator particularly effective.

The device 11 for measuring the air losses of the patient, which is present at the base of the second sealing chamber, is now described.

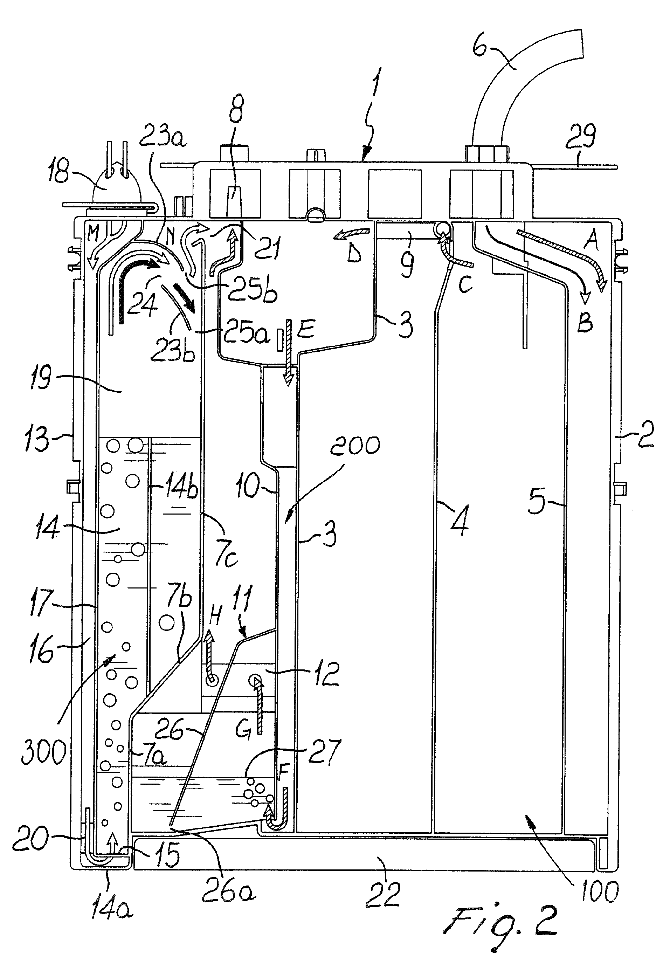

Device 11 comprises a bell 26, which has a passage port 26a at the lower end and is provided with the tap 12; at the base of the bell there is water, which in the normal operating conditions shown in Figure 2, with the tap 12 open and therefore such as to allow the passage of the air losses along the arrows G, H, maintains its free surface at a constant level 27.

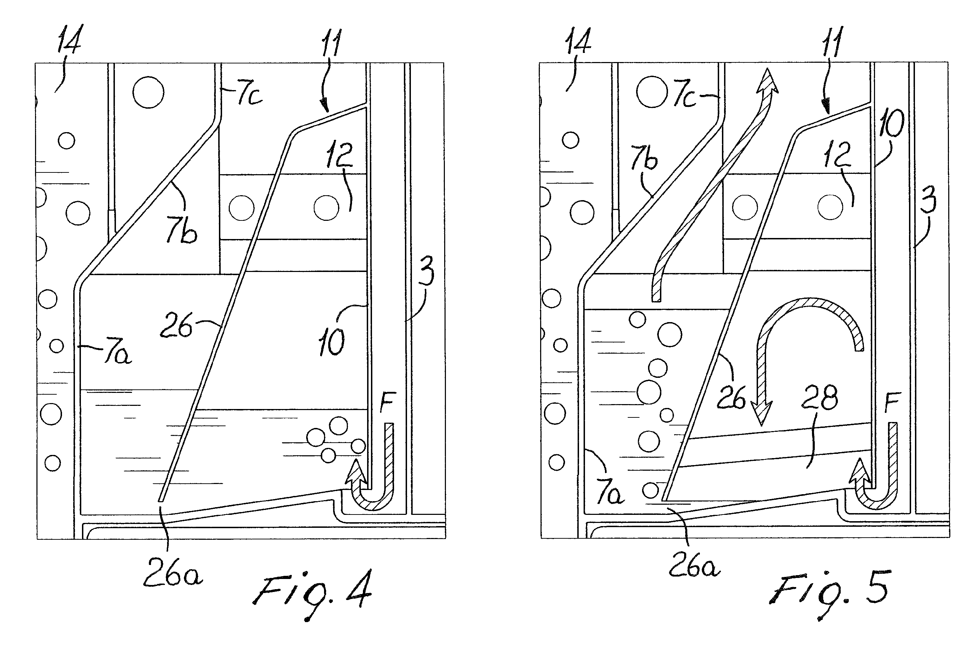

In order to measure the air losses, the tap 12 is closed, and so the air losses accumulate below the bell 26, gradually expelling the water through the port 26a, progressively providing situations of the type shown in Figure 4.

The measurement of the quantity of the losses is deduced from the time taken by the air losses to completely empty the bell of water, reaching the situation of Figure 5, and the importance for the operator of identifying the precise instant of the end of the emptying of the water from the bell is immediately evident.

For this purpose, a plane 28 that lies at the base of the bell has been given such an inclination as to make the region that directly faces the port 26a the lowest one.

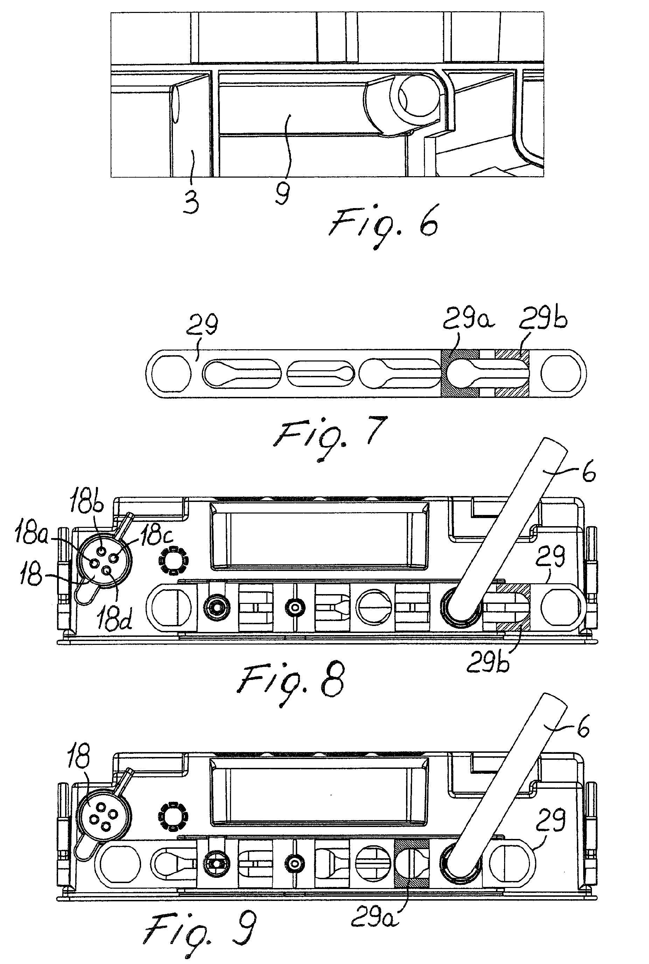

Considering in particular Figure 6, it is seen that the duct 9 for connection between the first chamber 100 and the second chamber 200 of the device is formed monolithically with the structure of the device, following the same design concept that provided monolithically also the bubbling partition 15 and substantially seeks to obtain the entire device with a single mold.

Finally attention is focused on a tube clamping plate 29, which is operated by the operator to obtain different functional conditions between the two extreme positions which are visible respectively in Figures 8 and 9.

In order to provide an indication which is immediately perceivable without any possibility of doubt regarding the assumed position, the plate 29 is provided with two regions 29a, 29b, which have different colorings and are designed to be selectively visible in the two extreme positions: thus, in the position of Figure 8, the region 29b is visible, and in the position of Figure 9 the region 29a is visible.

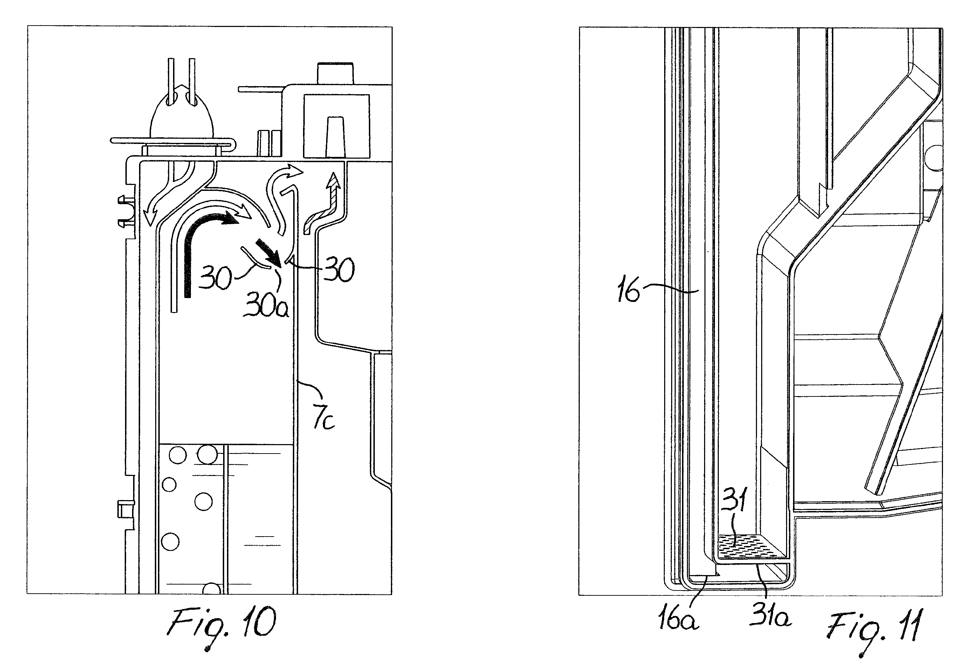

Figure 10 shows a further embodiment of the device according to the invention related to the phase separator described with reference to Figure 2; with respect to the previously described embodiment, the only variation consists in that the separation partition, i.e., the partition that in such embodiment was indicated by the reference numeral 23b, is provided here in the form of a cradle 30, which is connected to the wall 7c and has a port 30a for discharging the water, all the rest remaining unchanged.

Figure 11 shows another embodiment of the device according to the invention, which relates to the bubbling partition and to the methods for feeding atmospheric air thereto which are described with reference to Figure 3.

In this case, the bottom wall of the duct 16 that conveys the atmospheric air to a perforated bubbling partition 31 is flat and extends until it touches at 16a the bottom of the device, and thus the access of the air to the partition 31 occurs by means of a front side 31a; in this solution, the partition 31 has a differentiated calibration of the holes with a diameter which increases progressively as one moves away from the side 31a.

The described invention is susceptible of numerous other modifications and variations, all of which are within the scope of the appended claims; all the details may further be replaced with other technically equivalent elements.

The disclosures in Italian Patent Application no. MI2007A001649, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A thoracic draining device, comprising, in a monolithic structure, three interconnected contiguous chambers, which are adapted to be placed in partial vacuum by being connected to a source of suction which is capable of creating, within the device, a condition of vacuum which conveys away from the patient the exuded liquids and the air losses, said chambers comprising: a first chamber for collecting the exuded liquids, which is adapted to be connected by means of a tube to the thoracic cavity of a patient, so as to receive said exuded liquids and the air losses that exit from said cavity, a second sealing chamber, provided at the bottom with a device for measuring said air losses, a third chamber for adjusting the vacuum that occurs within the two preceding chambers, comprising a column of water through which atmospheric air is made to pass which enters at the bottom formed by a perforated bubbling partition and is conveyed thereat by a duct after entering through a perforated plug in order to be evacuated through a connector which is designed to be connected to the source of suction, **characterized in that** at the top of the third chamber, in said monolithic structure, a separator is provided for the two phases constituted by water and atmospheric air which are mixed in the stream that exits as a consequence of bubbling from the free surface of the water column that is present in said third chamber, with consequent fall of the water onto said column and exit of the atmospheric air from said chamber toward the suction source.

2. The device according to claim 1, **characterized in that** the separator of the two phases constituted by water and atmospheric air comprises at the top a baffle which is adapted to delimit, with an underlying partition, a duct which receives at the inlet the stream that comprises said phases mixed together and has a double exit port, one of said ports being adapted to cause the fall of the water toward the underlying column, the other port being adapted to send the atmospheric air toward the suction source.

3. The device according to one or more of the preceding claims, **characterized in that** it comprises, within said monolithic structure, means which are adapted to reduce noisiness, which comprise a particular shape of the end that lies proximate to the bubbling partition of the duct for conveying atmospheric air to said partition.

4. The device according to claim 3, **characterized in that** the end that lies proximate to the bubbling partition of the atmospheric air conveyance duct has a widening of said duct such as to allow the access of the atmospheric air to said bubbling partition at the transverse side thereof which is longer than the front side, the holes of the bubbling partition all having the same dimensions.

5. The device according to claim 4, **characterized in that** the bottom wall of the atmospheric air conveyance duct is, at the end located at the bubbling partition, contoured so as to form an inclined plane which widens said duct.

6. The device according to claim 3, **characterized in that** the bottom wall of the duct for conveying the atmospheric air to the bubbling partition is extended in a flat shape to the bottom of the device, so as to allow access of the atmospheric air to said bubbling partition at the front side thereof, a differentiated calibration of the holes of the bubbling partition, with a diameter which increases progressively as one moves away from said front side, being provided.

7. The device according to one or more of the preceding claims, **characterized in that** the bubbling partition is provided monolithically with the structure of said device.

8. The device according to one or more of the preceding claims, **characterized in that** the perforated plug for the inflow of the atmospheric air is provided with more than two holes.

9. The device according to one or more of the preceding claims, **characterized in that** the base of said device, at the column of water that is present in the third chamber, is arranged so as to be coplanar with the base of the support of said device.

10. The device according to one or more of the preceding claims, **characterized in that** the base plane of the bell comprised within the device for measuring air losses has such an inclination as to make the region that faces directly the port provided at the lower end of said bell the lowest region.

11. The device according to one or more of the preceding claims, **characterized in that** the duct for connection between the first chamber and the second chamber is provided monolithically with the structure of said device.

12. The device according to one or more of the preceding claims, comprising a tube clamping plate which is adapted to be actuated by an operator between two extreme positions, which correspond to different functional features, **characterized in that** said plate has two regions with different colors which are designed to be visible selectively in said extreme positions.

13. The device according to one or more of the preceding claims, **characterized in that** a single monolithic structure comprises all the parts which do not move with respect to each other and are potentially uniform in terms of material.
